# EUROPÄISCHE PATENTSCHRIFT

(11) **EP 0 847 375 B1**
(45) Veröffentlichungstag und Bekanntmachung des Hinweises auf die Patenterteilung: **11.09.2002**
(21) Anmeldenummer: 97925016.4
(22) Anmeldetag: 29.05.1997
(51) Int. Cl.: C03C 8/02, C03C 3/091, A61K 6/06

(54) **SILIKATGLASZUSAMMENSETZUNG UND VERFAHREN ZUR MODIFIKATION KERAMISCHER WERKSTOFFE**
SILICATE GLASS COMPOSITION AND PROCESS FOR MODIFICATION OF CERAMIC MATERIALS
COMPOSITION DE VERRE DE SILICATE ET PROCEDE DE MODIFICATION DE MATERIAUX CERAMIQUES

(30) Priorität: 29.05.1996 DE 19621357
(43) Veröffentlichungstag der Anmeldung: 17.06.1998
(73) Patentinhaber: Bonetec Holding Anstalt, 9493 Mauren (LI)
(72) Erfinder: VAN NIEKERK, Albert, D-65207 Wiesbaden (DE); MEYER, Leonhard, D-79618 Rheinfelden (DE)
(74) Vertreter: Kunz, Herbert, Dr.
(86) Internationale Anmeldenummer: EP9702800
(87) Internationale Veröffentlichungsnummer: WO97045377

(56) Entgegenhaltungen:
- EP-A- 0 478 937
- EP-A- 0 544 145
- WO-A-96/18373
- DE-A- 3 902 771
- DE-A- 3 911 460
- DE-A- 19 502 144
- US-A- 5 009 709
- DATABASE WPI Section Ch, Week 7818 Derwent Publications Ltd., London, GB; Class D21, AN 78-32979A XP002039590 & JP 53 031 716 A (WADA SEIMITSU-SHIKE) , 25.März 1978
- PATENT ABSTRACTS OF JAPAN vol. 013, no. 523 (C-657), 21.November 1989 & JP 01 212248 A (NIPPON ELECTRIC GLASS CO LTD), 25.August 1989,

## Beschreibung

Die Erfindung betrifft eine Silikatglaszusammensetzung zur Modifikation keramischer Werkstoffe, insbesondere Dentalwerkstoffe, sowie ein Verfahren zur Modifikation eines keramischen Werkstücks, vorzugsweise einer Dentalprothese. Außerdem betrifft die Erfindung die Verwendung der erfindungsgemäßen Silikatglaszusammensetzung.

Künstliche Zähne, wie sie beispielsweise für Kronen oder Brücken verwendet werden, können aus Porzellan hergestellt werden, das auf einen Metallgrundkörper aufgebrannt wird. Um ein möglichst naturgetreues Aussehen zu erreichen, wird das Porzellan in mehreren Schichten aufgebrannt, wobei auf das Metallgrundgerüst zunächst eine Schicht aus stark opakem Porzellan aufgebaut und gebrannt wird. Auf diese deckende Schicht wird dann eine weitere Schicht aus durchscheinendem Porzellan aufgetragen. Vor dem abschließenden Brennen kann noch eine weitere Schicht aus stärker durchscheinendem Porzellan als Schneidefläche aufgetragen werden, wodurch das natürliche Aussehen der künstlichen Zähne weiter verstärkt wird. Das Brennen des Porzellans erfolgt jeweils bei Temperaturen von ca. 650 - 1100 °C.

Um eine Anpassung an die Färbung der natürlichen Zähne zu erreichen, können dem Porzellan Pigmente beigefügt werden. In der DE 34 24 777 werden beispielsweise farbige Metalloxide vorgeschlagen, die als Färbekomponente einem Calciumphosphat-Glaskeramikmaterial beigegeben wird. Die Färbung wird durch eine Reaktion der Metalloxide mit dem keramischen Material erzeugt.

In der DE-OS-38 09 019 wird vorgeschlagen, den künstlichen Zahn schrittweise aufzubauen. Dabei werden dem durchscheinenden Keramikmaterial der einzelnen Schichten jeweils Pigmente zugemischt, und die Schicht anschließend gebrannt.

Nachteilig an diesen Färbemethoden ist, daß die Färbung der Keramik nach dem Brennen kaum noch verändert werden kann. Dies gilt insbesondere dann, wenn die Zahnfarbe zu dunkel ausgefallen ist. Eine Aufhellung der Keramik ist nach dem Brennvorgang praktisch nicht mehr möglich.

Ein weiteres Problem von keramischen Werkstoffen besteht darin, daß ihre mechanische Festigkeit um den Faktor 100 unter den theoretisch errechneten liegen. Ein Grund dafür sind die in der Griffithschen Bruchmechanik postulierten Mikrorisse. Sie sind mit bis zu 1000 Sprüngen pro cm² in Gläsern und Keramiken vorhanden und wegen ihrer Größe von ca. 3 - 6 µm ohne optische Hilfsmittel nicht sichtbar. Die Kerbstellenwirkung tritt verstärkt auf, wenn andere Materialien mit Gläsern oder Keramiken beschichtet werden. Durch unterschiedliche Wärmeausdehnung der Materialien treten zusätzliche Spannungen auf. Auch bei Temperaturwechseln mit geringem Temperaturunterschied können daher nach einer gewissen Zeit sogenannte Spätsprünge auftreten. Bei herkömmlichen Verfahren versucht man Materialien mit möglichst eng zusammenliegenden Wärmeausdehnungskoeffizienten zu verwenden und durch Temperatur-Zeit-Zyklen beim Brennen die Differenzen auszugleichen. Sind sichtbare Sprünge vorhanden oder ist die Stabilität des Werkstücks durch Mikrorisse zu stark reduziert, ist dieses unbrauchbar und als Ausschuß zu betrachten. Um brauchbare Ergebnisse zu erhalten, müssen viele verschiedene Parameter gleichzeitig beherrscht werden. Nur Personen mit viel Erfahrung erreichen einen zufriedenstellenden Qualitätsstandard. Zudem ist ein beträchtlicher Arbeits- und Geräteaufwand erforderlich, der die Kalkulation der Hersteller erheblich belastet.

Ein weiteres Problem bei der Herstellung künstlicher Zähne liegt in der Verbindung zwischen einem metallischen Grundgerüst und einer aus kosmetischen Gründen vorgesehenen Verblendung mit Kunststoff. Herkömmliche Verfahren ermöglichen nur eine "inselweise" Verbindung zwischen Kunststoff- und Metallflächen. In den Zwischenraum können daher Flüssigkeiten eindringen, wodurch die Verbindung geschwächt wird und Verfärbungen des Kunststoffes eintreten können.

Die WO-A-96 18 373 beschreibt ein niedrigschmelzendes Porzellan für Dentalanwendungen mit einem Schmelzpunkt von ca. 700°C ± 50°C und einem Wärmeausdehnungskoeffizenten von α = 12 - 13 µm K⁻¹. Das dort beschriebene Porzellan soll für Zahnprothesen in Kombination mit Metall- oder Porzellansubstraten verwendet werden.

Die JP-A-503 31 716 beschreibt eine Glasur für Dentalanwendungen, die Anteile von SiO₂, Al₂O₃, K₂O, Na₂O, B₂O₃, CaO und MgO enthält. Die beschriebene Glasur hat einen Wärmeausdehnungskoeffizienten von 125 - 160 x 10⁻⁷ / °C. Sie kann zur Beschichtung von metallischen Zahnkronen bei niedrigen Temperaturen von etwa 780°C bis 800°C verwendet werden.

In der EP-A-544 145 wird ein dentalkeramischer Werkstoff mit niedriger Verarbeitungstemperatur von 660°C ± 30°C beschrieben, der zur Herstellung und zur Rezeptur von metallkeramischem und vollkeramischem Zahnersatz dienen soll, der einen Wärmeausdehnungskoeffizienten von 13 - 14 x 10⁻⁶ K⁻¹ zwischen 20°C und 500°C und eine Verarbeitungstemperatur unterhalb von 700°C aufweisen soll. Er soll außerdem keine biologisch bedenklichen Bestandteile enthalten und im Mund korrosionsbeständig sein.

Aus der DE-A-39 11 460 ist ebenfalls eine niedrigschmelzende Dentalkeramik mit hoher Wärmedehnung und Korrosionsfestigkeit bekannt, bei der die Verarbeitungstemperatur bei ca. 720 °C liegt und gleichzeitig keine Nachteile in Bezug auf Wärmedehnung und/oder Korrosionsfestigkeit erreicht werden.

Keines der bekannten Keramikmaterialien ist aber bioaktiv.

Aufgabe der Erfindung ist es, eine Silikatglaszusammensetzung anzugeben, mit der die Materialeigenschaft bekannter Keramikmaterialien, insbesondere Dentalkeramiken, modifiziert werden können, sowie ein Verfahren zur Modifikation der Eigenschaften von Keramikmaterialien. Dabei soll sowohl eine Modifizierung bereits beim Aufbau der Keramik möglich sein bzw. die Eigenschaften einer bereits gebrannten Keramik noch nachträglich mit Hilfe der Zusammensetzung verändert werden können. Außerdem soll eine Bioaktivität der Keramik erreicht werden.

Erfindungsgemäß wird die Aufgabe mit einer Silikatglaszusammensetzung gelöst, die bezogen auf Gewichtsbasis im wesentlichen besteht aus

| | |
|---|---|
| SiO₂ | 35 - 65 % |
| Al₂O₃ | 7 - 15% |
| R₂O | 15 - 35% |
| R'O | 0 - 4 % |
| B₂O₃ | 0,5 - 12 % |
| ZrO₂ | 0 - 1 % |
| SnO₂ | 0 - 5% |
| TiO₂ | 0 - 5% |

wobei R ein oder mehrere Alkalimetalle, vorzugsweise Natrium oder Kalium und R' ein oder mehrere Erdalkalimetalle, vorzugsweise Calcium, ist, und die Silikatglaszusammensetzung einen Wärmeausdehnungskoeffizienten von α < 13 x 10⁻⁶ K⁻¹ aufweist. Die erfindungsgemäße Silikatglaszusammensetzung ist dadurch gekennzeichnet, daß sie durch Zusammenschmelzen der Oxide erhalten wird, nicht auf natürliche Mineralien zurückgeht, einen Transformationspunkt von Tₜ < 550°C, einen Erweichungspunkt von Tₑ < 600°C aufweist, und daß der Zusammensetzung ein Anteil von Hydroxilapatit, vorzugsweise von 10 - 40 % bezogen auf die Gesamtmasse beigemischt wird.

Die Zusammensetzung besitzt einen niedrigeren Schmelzpunkt und eine geringere Säurelöslichkeit im Vergleich zu mineralischen keramischen Werkstoffen vergleichbarer Zusammensetzung. Die Zusammensetzung geht nicht auf natürliche Mineralien zurück, die eventuell nach einer entsprechenden Reinigung, verarbeitet werden. Im Gegensatz zu diesen weist die Silikatglaszusammensetzung ein nichtstöchiometrisches Verhältnis der einzelnen Metalloxide auf und kann daher durch eine Variation des Anteils der einzelnen Komponenten individuell auf ein zu lösendes Problem angepaßt werden. Die Zusammensetzung bildet keine regelmäßige Kristallstruktur sondern liegt als Glas vor. Sie kann daher Spannungen, denen sie ausgesetzt wird, ausgleichen. So enthält die Zusammensetzung im Gegensatz zu mineralischen Keramiken beispielsweise keine, weniger oder kleinere Leucitkristalle. Durch Konzentrationsänderungen der Oxide in der Zusammensetzung, also der Netzwerkbildner und Netzwandler, sowie der sich amphoter verhaltenden Oxide, werden Eigenschaften, wie z.B. die Viskosität der Schmelze, die chemische Beständigkeit gegen Laugen und Säuren, die Wärmeausdehnung, der Brechungsindex sowie die Trübung beeinflußt werden. Durch das Temperatur-Zeitprogramm beim Erschmelzen der Zusammensetzung können Eigenschaften wie Transluzenz, Transparenz (Aufheizprogramm, Endtemperatur), Beständigkeit, Festigkeit, Kristallisation (Abkühlprogramm) und Zusammensetzung (Verdampfung leicht flüchtiger Anteile durch Haltezeit bei der Endtemperatur) gesteuert werden. Im Gegensatz zu keramischen Zusammensetzungen, die aus aufbereiteten Mineralien hergestellt werden und unter Umständen eine vergleichbare Zusammensetzung aufweisen können, zeigt die erfindungsgemäße Zusammensetzung überraschenderweise eine erhöhte Stabilität gegenüber Säuren. Mit herkömmlichen Keramiken läßt sich bei vergleichbaren physikalischen Eigenschaften, wie Transformationspunkt oder Wärmeausdehnungskoeffizient, eine vergleichbare Säurestabilität erreichen.

Die Silikatglaszusammensetzung ist durch die Beimischung des Anteils von Hydroxilapatit bioaktiv.

Die Eigenschaften der Silikatglaszusammensetzung sind besonders vorteilhaft, wenn die Zusammensetzung, bezogen auf die Gesamtmasse, als Alkalimetalloxid einen Anteil von 0 - 5 % Li₂O, 0 - 20 % K₂O, vorzugsweise 5 - 15 % K₂O, und/oder 10 - 30 % Na₂O aufweist.

Die Färbung, die Fluoreszenz bzw. die Opaleszenz der Keramik kann dadurch beeinflußt werden, daß der Zusammensetzung zumindest ein Pigment und/oder zumindest ein opaleszenzfördernder Stoff und/oder zumindest ein fluoreszenzfördernder Stoff beigemischt ist, vorzugsweise im Verhältnis von 20 : 1 bis 10 : 1 (v/v Zusammensetzung : Pigment).

Die Zusammensetzung läßt sich leicht verarbeiten, wenn die Zusammensetzung eine Pulverform oder Pastenform, vorzugsweise mit enger Korngrößenverteilung, aufweist.

Künstliche Zähne bzw. Dentalkeramiken mit hervorragenden Materialeigenschaften können erhalten werden, wenn der Zusammensetzung ein üblicher Keramikwerkstoff, vorzugsweise Dentalkeramikwerkstoff zugegeben ist, wobei bezogen auf die Gesamtmasse der Anteil der Silikatglaszusammensetzung 2 - 20 Gew.-%, insbesondere 5 - 10 Gew.-%, beträgt. Die Verarbeitung erfolgt wie bei den unmodifizierten keramischen Werkstoffen. Die Erfahrungen des Zahntechnikers können daher unmittelbar einfließen.

Das erfindungsgemäße Verfahren umfaßt die Schritte:

Anmischen einer Silikatglaszusammensetzung gemäß zumindest einem der Ansprüche 1 bis 5 mit einer farblosen, flüchtigen Flüssigkeit bis zu einer cremigen Konsistenz,
Auftragen einer dünnen Schicht der angemischten Zusammensetzung auf die gereinigte Oberfläche eines an sich fertig gebranntes keramisches Werkstücks; Brennen des beschichteten keramischen Werkstücks bei einer Temperatur von 650 - 950 °C, wobei die Brenntemperatur bis zu 180 °C unterhalb des Verglasungspunktes der für das Werkstück verwendeten Keramik liegt. Das Verfahren kann bei allen bekannten Keramiken, Gläsern und Glaskeramiken angewendet werden.

Bei dem erfindungsgemäßen Verfahren bleibt der Oberflächenglanz einer bereits gebrannten Keramik erhalten. Ein weiterer Glanzbrand ist daher nicht notwendig.

Durch das Brennen diffundiert die Zusammensetzung in die Oberfläche des bereits fertig gebrannten Werkstücks. Dabei können verschiedene Stoffe mit in das keramische Material eindiffundieren. So können beispielsweise Oberflächenfarben nachträglich in die Oberfläche des Werkstoffs eingebracht werden und somit eine Korrektur der Farbe erzielt werden. Insbesondere ist auch eine nachträgliche Aufhellung der Farbe eines künstlichen Zahnes bzw. einer Keramik erzielbar, was mit den bisher bekannten Methoden nicht möglich war. Ebenso können Opaleszenz und Fluoreszenz nachträglich verändert werden. Soll einer Keramik Bioaktivität verliehen werden, kann mit dem erfindungsgemäßen Verfahren auch nachträglich Hydroxylapatit auf der Oberfläche des künstlichen Zahns eingebracht werden.

Durch das erfindungsgemäße Verfahren werden auch Mikrorisse (Griffith-flaws) verschlossen. Solche unterhalb des Auflösungsvermögens des menschlichen Auges bleibenden Mikrorisse sind Ursache für Brüche und Sprünge, die erst nach einiger Zeit des Gebrauchs auftreten. Vorteilhaft führt daher auch eine Behandlung eines künstlichen Zahns bzw. eines keramischen Werkstücks mit einer farbangepaßten bzw. farbneutralen Silikatglaszusammensetzung zu einer Erhöhung seiner mechanischen Widerstandsfähigkeit.

Alternativ kann das Verfahren auch in der Weise durchgeführt werden, daß vor dem Auftragen der angemischten Zusammensetzung die Oberfläche des keramischen Werkstücks aufgerauht wird. Dies kann beispielsweise durch mechanische Bearbeitung oder durch Ätzen geschehen.

Ist die Oberfläche des keramischen Werkstücks nach dem Aufbrennen der Silikatglaszusammensetzung noch zu rauh, kann anschließend ein Glanzbrand durchgeführt werden, um eine bessere Körperverträglichkeit zu erreichen.

Insbesondere bei bioaktiven Keramiken kann die Oberfläche nach dem Brennen auch angeätzt werden.

Die Eigenschaften der Oberfläche lassen sich auch durch Variationen des Brennvorgangs beeinflussen. Es hat sich als günstig erwiesen, den Brennvorgang mit einem Temperaturprogramm durchzuführen. Dabei wird insbesondere zunächst bei ca. 300 - 400 °C die Silikatglaszusammensetzung getrocknet und anschließend das Werkstück mit einer Heizrate von ca. 50 - 70 °C/min bis auf Brenntemperatur erwärmt.

Vorteilhaft kann die erfindungsgemäße Silikatglaszusammensetzung auch als Grundierungsmittel für Verbindungen zwischen metallischen Werkstücken und solchen aus Kunststoff verwendet werden. Die Zusammensetzung läßt sich nahezu spannungsfrei auf ein metallisches Grundgerüst aufziehen. Nach Aufbringen eines Silans kann dann eine Haftverbindung, beispielsweise zu einem Kunststoffwerkstück, hergestellt werden. Diese kann so ausgeführt werden, daß die gesamte Kontaktfläche zwischen metallischem Grundgerüst und Kunststoffkörper als Haftfläche wirkt. Insbesondere bei Zahnprothesen kann dann keine Flüssigkeit zwischen die beiden Werkstücke eindringen, wodurch die Beständigkeit der Verbindung erhöht wird.

## Patentansprüche

1. Silikatglaszusammensetzung zur Modifikation keramischer Werkstoffe bezogen auf Gewichtsbasis im wesentlichen bestehend aus:
| | |
|---|---|
| SiO₂ | 35 - 65 % |
| Al₂O₃ | 7 - 15 % |
| R₂O | 15 - 35 % |
| R'O | 0 - 4% |
| B₂O₃ | 0,5 - 12 % |
| ZrO₂ | 0 - 1 % |
| SnO₂ | 0 - 5% |
| TiO₂ | 0 - 5% |
wobei R ein oder mehrere Alkalimetalle, vorzugsweise Natrium oder Kalium,
und R' ein oder mehrere Erdalkalimetalle, vorzugsweise Calcium, ist,
die Silikatglaszusammensetzung einen Wärmeausdehnungskoeffizienten von α < 13 × 10⁻⁶ K⁻¹ aufweist,
**dadurch gekennzeichnet, daß**
die Silikatglaszusammensetzung durch Zusammenschmelzen der Oxide erhalten wird,
nicht auf natürliche Mineralien zurückgeht,
einen Transformationspunkt von Tₜ < 550 °C
und einen Erweichungspunkt von Tₑ < 600 °C aufweist,
und daß der Zusammensetzung ein Anteil von Hydroxilapatit, vorzugsweise von 10 - 40 % bezogen auf die Gesamtmasse, beigemischt wird.

2. Zusammensetzung nach Anspruch 1, **dadurch gekennzeichnet, daß** die Zusammensetzung, bezogen auf die Gesamtmasse, als Alkalimetalloxid einen Anteil von 0-5 % Li₂O, 0-20 % K₂O, vorzugsweise 5-15 % K₂O und/oder 10-30 % Na₂O aufweist.

3. Zusammensetzung nach Anspruch 1 oder 2, **dadurch gekennzeichnet, daß** der Zusammensetzung zumindest ein Pigment und/oder zumindest ein opaleszensfördernder Stoff und/oder zumindest ein fluoreszenzfördernder Stoff beigemischt ist, vorzugsweise im Verhältnis von 20:1 bis 10:1 (v/v, Zusammensetzung : Pigment).

4. Zusammensetzung nach Anspruch 1, 2 oder 3, **dadurch gekennzeichnet, daß** die Zusammensetzung eine Pulverform oder eine Pastenform, vorzugsweise mit enger Komgrößenverteilung, aufweist.

5. Zusammensetzung nach Anspruch 1, 2, 3 oder 4, **dadurch gekennzeichnet, daß** der Zusammensetzung ein üblicher Keramikwerkstoff zugegeben ist, wobei bezogen auf die Gesamtmasse der Anteil der Zusammensetzung vorzugsweise 2 - 20 Gew.-% insbesondere 5 - 10 Gew.-%, beträgt.

6. Verfahren zur Modifikation eines keramischen Werkstücks, vorzugsweise einer Dentalprothese, **gekennzeichnet durch** die Schritte:
Anmischen einer Silikatglaszusammensetzung gemäß zumindest einem der Ansprüche 1 - 5 mit einer farblosen, flüchtigen Flüssigkeit bis zu einer cremigen Konsistenz,
Auftragen einer dünnen Schicht der angemischten Zusammensetzung auf die gereinigte Oberfläche eines an sich fertig gebrannten keramischen Werkstücks;
Brennen des beschichteten keramischen Werkstücks bei einer Temperatur von 650°C - 950°C, wobei die Brenntemperatur bis zu 180°C unterhalb der Verglasungstemperatur der für das Werkstück verwendeten Keramik liegt.

7. Verfahren nach Anspruch 6, **dadurch gekennzeichnet, daß** vor dem Auftragen der angemischten Zusammensetzung die Oberfläche des keramischen Werkstücks aufgeraut wird.

8. Verfahren nach Anspruch 6 oder 7, **dadurch gekennzeichnet, daß** nach dem Beschichten des keramischen Werkstücks mit der Silikatglaszusammensetzung ein Glanzbrand durchgeführt wird.

9. Verfahren nach Anspruch 6, 7 oder 8, **dadurch gekennzeichnet, daß** nach dem Brennen die Oberfläche des Werkstücks angeätzt wird.

10. Verfahren nach Anspruch 6, 7, 8 oder 9, **dadurch gekennzeichnet, daß** das Brennen mit einem Temperaturprogamm erfolgt, insbesondere indem zunächst bei ca. 300°C - 400°C getrocknet wird und anschließend mit einer Heizrate mit ca. 50°C - 70°C / min bis auf Brenntemperatur erwärmt wird.

11. Verwendung einer Silikatglaszusammensetzung gemäß einem der Ansprüche 1 bis 5 als hochfestes Grundierungsmittel, vorzugsweise für Verbindungen zwischen Metall- und Kunststoffwerkstücken.

## Claims

1. Silicate-glass composition for modification of ceramic materials with reference to weight basis essentially consisting of:
| | |
|---|---|
| SiO₂ | 35-65% |
| Al₂O₃ | 7-15% |
| R₂O | 15-35% |
| R'O | 0-4% |
| B₂O₃ | 0,5-12% |
| ZrO₂ | 0-1% |
| SnO₂ | 0-5% |
| TiO₂ | 0-5% |
where R is one or more alkali metals, preferably sodium or potassium, and R' is one or more alkaline earth metals, preferably calcium, the silicate-glass composition shows a coefficient of thermal expansion of α < 13 x 10⁻⁶ K⁻¹,
**characterized in that**
the silicate-glass composition is obtained by fusing the oxides,
is not derived from natural minerals,
shows a transformation point of Tₜ < 550 °C
and a softening point of Tₑ < 600 °C
and that a portion of hydroxilapatite, preferably 10 - 40 % of the overall mass, is added to the composition.

2. Composition in accordance with claim 1, **characterized in that** the composition, with reference to the overall mass, shows as alkali metal oxide, a portion of 0 - 5 % Li₂O, 0 - 20 % K₂O, preferably 5 - 15 % K₂O and/or 10 - 30 % Na₂O.

3. Composition in accordance with claim 1 or 2, **characterized in that** at least one pigment and/or at least one opalescence-supporting substance and/or at least one fluorescence-supporting substance is added to the composition, preferably in a proportion of 20 : 1 to 10 : 1 (v/v, composition: pigment).

4. Composition in accordance with claim 1, 2 or 3, **characterized in that** the composition has the form of powder or paste, preferably with narrow grain size distribution.

5. Composition in accordance with claim 1, 2, 3, or 4, **characterized in that** a common ceramic material is added to the composition, with the portion of the composition, regarding the overall mass, preferably being 2 - 20 weight-%, in particular 5 - 10 weight-%.

6. Method for modification of a ceramic work piece, preferably of a dental prosthesis, **characterized by** the steps of:
Mixing a silicate-glass composition in accordance with at least one of claims 1 - 5, with a colourless, volatile fluid up to a creamy consistence;
Applying a thin coat of the mixed composition onto the cleaned surface of a principally finished fired ceramic work piece;
Firing the coated ceramic work piece at a temperature of 650 °C - 950 °C, with the firing temperature being up to 180 °C below the glazing temperature of the ceramic used for the work piece.

7. Method in accordance with claim 6, **characterized in that** the surface of the ceramic work piece is roughened before applying the mixed composition onto.

8. Method in accordance with claim 6 or 7, **characterized in that** a bright firing is performed after coating the ceramic work piece with the silicate-glass composition.

9. Method in accordance with claim 6, 7 or 8, **characterized in that** the surface of the working piece is etched after firing.

10. Method in accordance with claim 6, 7, 8 or 9, **characterized in that** the firing is effected by a temperature program, particularly by firstly drying at approx. 300 °C - 400 °C and then by heating up to firing temperature with a heating rate of approx. 50 °C - 70 °C / min.

11. Use of a silicate-glass composition in accordance with any of claims 1 to 5 as high-stable primer material, preferably for connections between metallic and plastic work pieces.

## Revendications

1. Composition de verre de silicate pour modification de matériaux céramiques par rapport à la masse totale, principalement consistant en:
| | |
|---|---|
| SiO₂ | 35 - 65 % |
| Al₂O₃ | 7 - 15 % |
| R₂O | 15 - 35 % |
| R'O | 0 - 4 % |
| B₂O₃ | 0,5 - 12 % |
| ZrO₂ | 0 - 1 % |
| SnO₂ | 0 - 5% |
| TiO₂ | 0 - 5 % |
R étant un ou plusieurs métaux alcalis, préférablement le sodium ou le kalium,
et R' étant un ou plusieurs métaux terrestres alcalis, préférablement le calcium,
la composition de verre de silicate comportant un coefficient d'expansion thermique de α < 13 x 10⁻⁶ K⁻¹,
**caractérisée en ce que**
la composition de verre de silicate est obtenue par fusion d'oxydes,
n'est pas une dérivée de minéraux naturels possède,
compose un point de transformation Tₜ < 550 °C
et un point de fusion Tₑ < 600 °C,
et qu'une partie d'hydroxilapatite, préférablement 10 - 40 % de la masse totale, est ajouté à la composition.

2. Composition selon la revendication 1, **caractérisée en ce que** la composition, par rapport la masse totale, comporte comme oxyde de métal alcali une partie de 0 - 5 %, Li₂O, 0 - 20 % K₂O, préférablement 5 - 15 % K₂O et/ou 10 - 30 % Na₂O.

3. Composition selon la revendication 1 ou 2, **caractérisée en ce qu'**au moins un pigment et/ou au moins une substance supportant l'opalescence et/ou au moins une substance supportant la fluorescence est ajoutée à la composition, préférablement en proportions 20 : 1 à 10 : 1 (v/v, composition: pigment).

4. Composition selon la revendication 1, 2 ou 3, **caractérisée en ce que** la composition comporte une forme de poudre ou une forme pâteuse, préférablement avec une distribution étroite de taille/grandeur de grain.

5. Composition selon la revendication 1, 2, 3 ou 4, **caractérisée en ce qu'**un matériau céramique courant est ajouté à la composition, la partie de la composition étant préférablement 2- 20 %, en particulier 5- 10 % du poids, par rapport la masse totale.

6. Procédé de modification d'un matériau en céramique, préférablement d'un dentier,
**caractérisée par:**
Mixage d'une composition de verre de silicate selon au moins une des revendications 1 - 5, avec un liquide incolore et volatil jusqu'à une consistance onctueuse;
Application d'une fine couche de la composition mixée sur la surface nettoyée d'une pièce / un élément en céramique principalement cuite;
Cuisson de la pièce / élément en céramique recouvert(e) à une température de 650 °C - 950 °C, la température de cuisson étant jusqu'à 180 °C au-dessous de la température de vitrage de la céramique utilisée pour la pièce / l'élément.

7. Méthode selon la revendication 6, **caractérisée en ce que** la surface de la pièce / de l'élément en céramique est rendue rude avant l'application de la composition mixée.

8. Méthode selon la revendication 6 ou 7, **caractérisée en ce qu'**une cuisson brillante est effectuée après recouvrance de la pièce / de l'élément en céramique avec la composition de verre de silicate.

9. Méthode selon la revendication 6, 7 ou 8, **caractérisée en ce que** la surface de la pièce / de l'élément est corrodée après la cuisson.

10. Méthode selon la revendication 6, 7, 8 ou 9, **caractérisée en ce que** la cuisson est effectuée avec un programme de température, en particulier par d'abord séchant à environ 300 °C- 400 °C et après chauffant avec un taux de chauffage de 50 °C - 70 °C / min jusqu'à la température de cuisson.

11. Utilisation d'une composition de verre de silicate selon une des revendications 1 à 5 comme matériau de sous-couche de haute ténacité, préférablement pour connexions entre des pièces / éléments en métal et en plastique.
